# EUROPEAN PATENT APPLICATION

(11) **EP 4 742 260 A1**
(43) Date of publication of application: **13.05.2026**
(21) Application number: 24211024.5
(22) Date of filing: 06.11.2024
(51) Int. Cl.: G16H 20/30, G16H 50/30, A61B 5/11

(54) **A METHOD AND A SYSTEM FOR REAL-TIME MOVEMENT AND PERFORMANCE ANALYSES FOR A HUMAN**

(71) Applicant: Kiso ehf., 105 Reykjavík (IS)
(72) Inventor: THORGILSSON, Baldur, 105 Reykjavík (IS)
(74) Representative: HKW Intellectual Property PartG mbB

(57) **Abstract**

The present invention relates to a method and a system for real-time movement and performance analysis for a human using an external computer system and at least one wireless mobile monitoring device, where the monitoring device measures articular angle and/or muscular activity of an object part while the human moves the object part in accordance with pre-defined moving instructions. By processing this data, a real-time movement performance analysis indicator may be calculated indicating if the muscular activity for the muscular object is as expected or not.

## Description

### FIELD OF THE INVENTION

The present invention relates to a system and a method for real-time movement and performance analysis for a human.

### BACKGROUND OF THE INVENTION

Musculoskeletal problems are among the largest health issues in the world, and the number of people requiring solutions is growing faster than healthcare providers can manage. Musculoskeletal problems are one of the leading causes of pain and reduced mobility globally, making them a particular focus for the World Health Organization, WHO. The result is that people live with these ailments longer than necessary, leading to associated negative social impacts and costs. Up to one-third of the world's population suffers from musculoskeletal problems, with the percentage being higher in developed countries.

Physical therapists and trainers help their clients to find the best exercises. Their typical service is based on experience, knowledge from books, and/or courses rather than direct measurements. The field is characterized by subjective assessments of which exercises yield the best results. Measurements have shown that the exercises clients are given do not always produce the expected results.

Physical therapists are likely the professional group most knowledgeable about human movement. They have a solid foundational knowledge but often lack the time or resources to utilize results from the scientific research and therefore don't have good and standardized guidelines on how to use existing equipment's such as build into the instructions, similar to the movement guidelines. It is estimated that about 70% of their work involves influencing muscles. Measurements typically expected in a standard physical therapy setting are often limited to questionnaires, goniometers, or a single video recording of movement. Thus, a typical physical therapy clinic rarely engages in objective measurements of movement ability. Often patients suffer from lack of muscle control and muscle imbalance which manifests in such a way that one muscle takes over the function of another or is inactive or hyperactive without changing behavior of other muscles. This issue often goes undetected by traditional methods, leading to weeks or even months of treatment on a specific muscle without achieving the expected results.

### SUMMARY OF THE INVENTION

It is an object of the invention to address the identified challenges by providing a clinically fitting system and method capable of accurately assessing and intervene musculoskeletal issues, where objective measurements and insights into e.g. muscle control and imbalances, which are often overlooked by traditional approaches may be solved.

In general, the invention preferably seeks to mitigate, alleviate or eliminate one or more of the above-mentioned disadvantages of the prior art singly or in any combination. In particular, it may be seen as an object of embodiments of the present invention to provide precise musculoskeletal related data as input in enhancing the effectiveness of treatments and to ensure that patients receive more targeted and scientifically backed interventions, leading to better outcomes and reduced treatment times.

In a first aspect of the invention, a system is provided for real-time movement and performance analysis for a human, comprising:
- at least one wireless mobile monitoring device configured to be placed on an object part of the human, comprising:
   ∘ a communication module,
   ∘ an orientation sensor, and/or
   ∘ a muscular activity sensor,
- a computer system configured to present pre-stored moving instructions to the human,

where the orientation sensor and/or the muscular activity sensor is/are configured to acquire articular angle data and/or muscular activity data of the object part while the human moves the object part in accordance with the moving instructions,
where the articular angle position data and/or the muscular activity data is/are communicated by the communication module to the computer system where the data is processed, where the processing includes comparing the received articular angle position data and/or the muscular activity data with reference data and determining a real-time movement and performance analysis indicator for the object part.

The computer system may in an embodiment comprise a computer device comprising a device receiver, a display and a device processor for controlling the device receiver and the display. The computer device may as an example comprise a stationary computer, or any type of a portable device such as a tablet device, mobile phone and the like. The device receiver is configured for receiving the data communicated by the communication module and the display is in an embodiment configured to display the moving instructions via the display. The communication of the data via the communication module to the device receiver may be done via communication protocol such as, but not limited to, WIFI or Bluetooth. The computer device may also comprise a memory for storing the received data, which may be beneficial in case the communication is not present or the signal is bad.

In an embodiment, the computer system further comprises a computer platform comprising a platform memory, a platform transceiver and a platform processor for controlling the platform memory and the platform transmitter. The moving instructions may be stored by the platform memory, and the platform processor may be configured to instruct the platform transceiver to transmit the stored moving instructions to the computer device where the moving instructions are stored in a device memory comprised in the computer device. This may as an example be done via a Transmission Control Protocol/Internet Protocol (TCP/IP) protocol. The transmitted stored moving instructions may in another alternative embodiment be displayed in real-time at the computer device via the display.

In an embodiment, the orientation sensor comprises an Inertial Measurement Unit (IMU) sensor or Magnetic Angular Rate and Gravity (MARG) sensor.

Using IMU/MARG is suitable when the orientation is of relevance, but these sensors can also measure acceleration and the magnetic field, which might be of interest, for example, for automatically detecting steps. Therefore, it might be worth mentioning that we are also using the motion sensors to record acceleration, for example for impact detection, and to make angle measurement more accurate. Thus, the acceleration may also be measured for e.g. impact detection, to ensure more accurate articular angle detection.

In an embodiment, the muscular activity sensor comprises an Electromyography (EMG) sensor, such as surface Electromyography (EMG) sensor and where the muscular activity data is a surface or non-surface muscular activity data.

In an embodiment, the step of processing the articular angle position data and/or the muscular activity data includes processing both the articular angle position data and the muscular activity data, preferably simultaneously. The processing may be performed by a device processor comprised in the wireless mobile monitoring device(s) or by the platform processor. The processing comprises:
- utilizing the articular angle data to align the muscular activity data with corresponding muscular activity reference data for each acquired articular angle position measured by the orientation sensor, and
- utilizing the resulting muscular activity data pair for each acquired articular angle position measured by the orientation sensor in calculating the real-time movement and performance analysis indicator for the muscular object part.

The system enables, while performing the above-mentioned movements and calculations in real-time while the human is performing the moving instructions/exercises, acquiring the necessary data needed as input data to make an individualized training program and determine the exercises that maximizes the result of the training for the human when handling musculoskeletal problems and shortens the treatment time.

The device or platform processor may in an embodiment further be configured to present in real-time the measured muscular activity data and/or the muscular activity reference-data. This may as an example be done via the display where the human and/or any physical therapists or medical expert may visually see in real-time the muscular activity compared to target activity, where the human and/or the medical expert may act on it accordingly, e.g. by changing the movements if there is too much deviation between the muscular activity compared to target activity indicated by the performance analysis indicator.

As will be discussed in more details later, multiple of the wireless mobile monitoring device may be placed on the object part during the above-mentioned processing, such as placing a wireless mobile monitoring device on a healthy object part and corresponding injured object part, e.g. right and left shoulder, right and left arm, right and left knee etc., where the object part "pairs" may be moved simultaneously, or individually.

As mentioned above, in some instances, humans suffer from lack of muscle control and muscle imbalance which manifests in such a way that one muscle takes over the function of another. Causes of muscle imbalance can be, for example, after an accident or strain. As a result, the current exercises the human is doing may not improve this condition at all, resulting in that pain persists, as well as restricted joint motion. Prolonged imbalance can lead to joint wear and tear, among other issues, which eventually may require surgical intervention.

Thus, by measuring the muscular activity of more than one muscular object part, it is possible to identify in real-time via the performance analysis indicator if one muscle has "taken over" another muscle for which the muscular training program is designed for to build up the muscular activity. This may as an example be based on if the deviation is above or below a pre-defined threshold, the performance analysis indicator may indicate this via any type of a command, sound or visually to the human or physiotherapist.

Based on the above, a compact and user-friendly system is provided for such a movement and performance analysis that does not e.g. require spacious area.

In a second aspect of the invention, a method is provided of real-time movement and performance analysis for a human, where the method comprises:
- providing a pre-stored moving instructions characteristic for an object part of the human,
- presenting the moving instructions to the human,
- providing at least one wireless mobile monitoring device configured to be placed on the object part of the human, comprising:
   ∘ a communication module,
   ∘ an orientation sensor, and/or
   ∘ a muscular activity sensor,
- measuring, by the orientation and/or the muscular activity sensor, the articular angle data and/or muscular activity of the object part while the human moves the object part in accordance with the moving instructions,
- communicating by the communication module the measured articular angle data and/or the muscular activity to a computer system where the data is processed, where the processing includes,
- comparing the received articular angle position data and/or the muscular activity data with reference data and determining a real-time movement and performance analysis indicator for the object part.

The step of processing the received articular angle position data and/or the muscular activity data includes processing both the articular angle position data and the muscular activity data simultaneously. This may be performed by means of utilizing the articular angle data to align the muscular activity data with corresponding muscular activity reference data for each acquired articular angle position measured by the orientation sensor. That means as an example that for a given articular angle data a and muscular activity data M₁ pair (a,M₁) a third dimension data point is added, namely M₂ resulting in (a,M₁, M₂)ᵢ where M₂ is the muscular activity reference data for the articular angle data a., where tens hundreds or thousands of such datapoints (a,M₁, M₂)ᵢ are then be presented in real-time via any type of output means such as a display.

This reference data may in an alternative embodiment origin from a pre-stored reference data base where the data indicate how a healthy muscular activity data should be, or it may origin from a corresponding healthy object part of the human.

The term object parts may according to the present invention be understood as a muscular object parts and/or non-muscular object part.

In an embodiment, the method further comprises, initially, in response to selecting an object part or object parts on the human to be analyzed, providing information indicating where to place the orientation and/or the muscular activity sensor on the human. These instructions may as an example be presented in a similar way to the movement instructions for the patient.

Obviously, depending on which object part to be analyzed, e.g. shoulder vs. leg, the placement of the orientation and/or the muscular activity sensor(s) is highly dependent thereon. In that way, it is ensured that the orientation and/or the muscular activity sensor(s) are placed on the correct object parts before the measurements start.

In an embodiment, multiple of wireless mobile monitoring devices are utilized to be placed on an injured object part (first object part) and corresponding healthy object part (second object part) on the human, which may be the same object part, where the reference data comprise the data measured on the corresponding healthy object. This may include a scenario where both object parts are moved simultaneously, or not simultaneously, e.g. where the healthy object part is first moved, and then the injured object part, or vice versa. In some scenarios the first and the second object parts are not the same object parts.

An example of a muscular object part is Biceps Brachii, Pectoralis Major, Rectus Abdominis, Quadriceps Femoris, Deltoid, Gastrocnemius, Gluteus Maximus, Latissimus Dorsi, just to mention few muscular object parts.

In an alternative embodiment, the orientation sensor may be placed on a non-muscular object part while measuring the articular angle data of the object part.

In an embodiment, the step of calculating the real-time movement and performance analysis indicator for the object part comprises calculating the difference between the muscular activity data and the corresponding muscular activity reference data for each acquired articular angle position measured by the orientation sensor, where if the difference is above or below a pre-defined threshold biofeedback command or information is issued. The biofeedback command may as an example include issuing moving instructions at least partly different from the pre-stored moving instructions, but this may be done visually via the display, via speech command, or simply move the object part in a different way. This may in an alternative embodiment, be continued until the difference between the first muscular activity data and the corresponding muscular activity reference data is above or below the pre-defined threshold.

In an embodiment, the step of calculating the real-time movement and performance analysis indicator for the object part comprises calculating the difference between the muscular activity data and the corresponding muscular activity reference data for each acquired articular angle position measured by the orientation sensor, where if the difference is above or below a pre-defined threshold biofeedback command is issued. The biofeedback command may as an example comprise issuing moving instructions at least partly different from the pre-stored moving instructions. This may in an embodiment be repeated until the difference between the first muscular activity data and the corresponding muscular activity reference data is above or below the pre-defined threshold. In that way, it is possible to adjust the movement of the object part via the biofeedback command until "sufficient" muscular activity is present and sufficient to build up the first muscular object part. In that way, it is possible to identify in real-time if the moving instructions characteristic for the muscular object are maximizing the result of the training for the human and changes in the movements may be proposed until the most optimal result is obtained.

In general, the various aspects of the invention may be combined and coupled in any way possible within the scope of the invention. These and other aspects, features and/or advantages of the invention will be apparent from and elucidated with reference to the embodiments described hereinafter.

### BRIEF DESCRIPTION OF THE DRAWINGS

Embodiments of the invention will be described, by way of example only, with reference to the drawings, in which
Figure 1 shows an embodiment of a system according to the present invention for real-time movement and performance analysis for a human,
Figure 2 shown a flowchart of a method according to the present invention method of real-time movement and performance analysis for a human,
Figure 3 depicts graphically where a human follows moving instructions shown by a silhouette on a laptop, tablet, mobile phone,
Figure 4 shows the embodiment in figure 3 but where the moving instructions are given orally by a physical therapist or a medical expert,
Figure 5 shows an embodiment where a second wireless mobile monitoring device, comprising a second transmitter, a second orientation sensor and a second muscular activity sensor is placed on the human,
Figure 6 shows another scenario where the three wireless monitoring devices and are being utilized on each arm, and
Figure 7 shows an exemplary embodiment where the human starts with moving the right healthy arm with the second wireless mobile monitoring device in accordance to the moving instructions as discussed in relation to figures 3 and 4.

### DESCRIPTION OF EMBODIMENTS

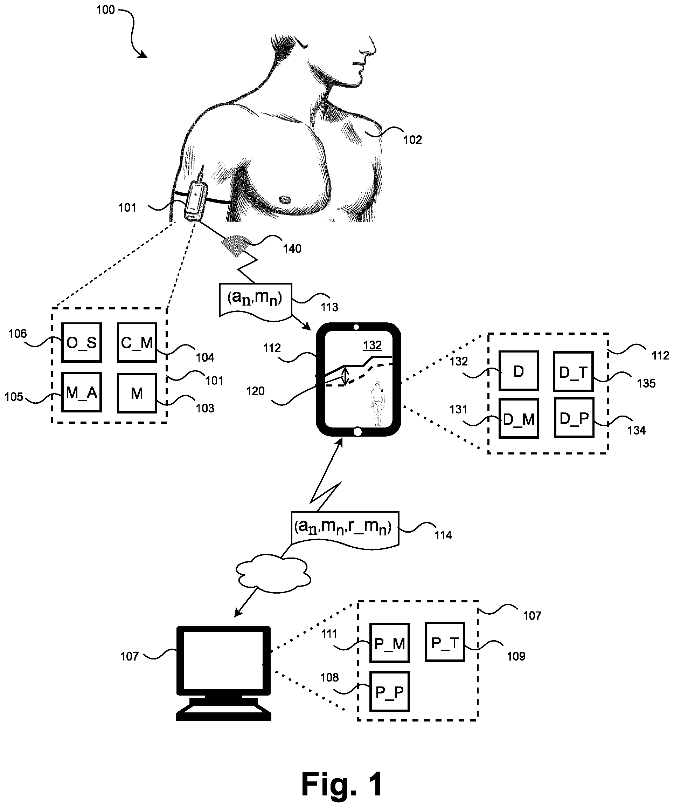

Figure 1 shows an embodiment of a system 100 according to the present invention for real-time movement and performance analysis for a human 102, where the system comprises a wireless mobile monitoring device 101, a computer device 112 and an external computer platform 107.

The first wireless mobile monitoring device 101 is attached, e.g. via strip or adhesive electrodes, to an object part of the human 102 which may be a muscular object part or non-muscular object part. The monitoring device comprises a communication module (C_M) 104, an orientation sensor (O_S) 106, a memory 103 and a muscular activity sensor (M_A) 105.

The orientation sensor may in an embodiment comprise an Inertial Measurement Unit (IMU) sensor or Magnetic Angular Rate and Gravity (MARG) sensor and the muscular activity sensor may in an embodiment comprise a surface ElectroMyoGraphy (EMG) sensor and where the muscular activity data is a surface muscular activity data where the EMG sensor preferably comprises at least two electrodes.

In the embodiments discussed in relation to the figures, both the articular angle position data aₙ and the muscular activity data mₙ is measured simultaneously and where the data pair (aₙ, mₙ) 113 is communicated in real time by the communication module 104 via communication protocol such as WIFI or Bluetooth 140 to the computer device 112. In the embodiment illustrated here, the data is forwarded to the external platform computer 107 where the data is processed. These data may also be stored in the memory 103, which may be beneficial in case the communication is not present or the signal is bad.

It should be noted that in another alternative embodiment the data may be processed and displayed at the computer device 112, i.e. where the processing steps illustrated here are performed by the computer device 112.

The processing includes comparing the received articular angle position data and the muscular activity data with reference data and determining a real-time movement and performance analysis indicator for the object part.

The computer device 112 may be any type of portable computer such as a tablet computer, mobile phone and the like comprising a device transceiver (D_T) 135, a display (D) 132, a device memory (D_M) 131 and a device processor (D_P) 134 for controlling the device receiver, the display and the device memory. The device transceiver 135 is configured for receiving the data 113 communicated by the communication module 104 and the display 132 is configured to display the above-mentioned moving instructions.

The external platform computer 107 comprises a platform memory (P_M) 111, a platform transceiver (P_T) 109 and a platform processor (P_P) 108 for controlling the platform memory and the platform transceiver. The moving instructions may be stored in the platform memory, and the platform processor may be configured to instruct the platform transceiver to transmit the stored moving instructions to the computer device 112 where the moving instructions may subsequently be stored in the device memory 131. The step of transmitting may as an example be done via a Transmission Control Protocol/Internet Protocol (TCP/IP) protocol. The transmitted stored moving instructions may in another alternative embodiment be displayed in real-time at the computer device 112 via the display 132.

Processing the data pair (aₙ, mₙ) 113 includes utilizing the first articular angle data to align the first muscular activity data with corresponding muscular activity reference data r_mₙ for each acquired articular angle position measured by the first orientation sensor, and utilizing the resulting data point (aₙ, mₙ, r_mₙ) for each acquired articular angle position measured by the first orientation sensor in calculating a real-time movement and performance analysis indicator for the first muscular object part.

The external computer system 107 is further configured to transmit the processed data (aₙ, mₙ, r_mₙ) 114 to the computer device 112 where the data points and the performance analysis indicator may be presented in real-time for the user. The performance analysis indicator may in the embodiment illustrated here reflect a deviation of the first muscular activity data from the muscular activity reference-data as indicated by the arrow 120.

As mentioned before, the processing of the data may be done at the computer device 112 side and presented in real time. This data may be sent to the external platform computer 107 where the data is amongst others stored.

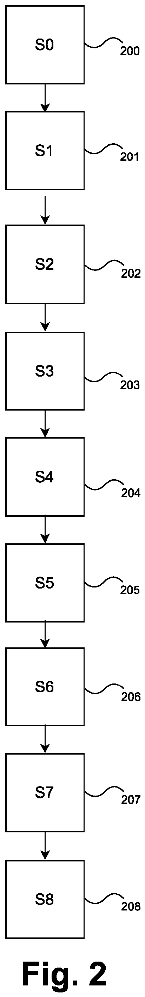

Figure 2 shown a flowchart of a method according to the present invention method of real-time movement and performance analysis for a human.

In step (S0) 200, before it comes to the movement, a medical or physical therapists needs to know how to set up the equipment, i.e. which equipment goes where. These instructions may as an example be presented in a similar way to the movement instructions for the patient discussed here below.

In step (S1) 201, pre-stored moving instructions is provided characteristic for an object part of the human and presented to the human and/or medical or physical therapists.

In step (S2) 202, a first wireless mobile monitoring device is provided configured to be placed on the object part of the human, which may be a muscular or non-muscular object part.

In step (S3) 203, the articular angle position of the object part is measured by the orientation sensor while the human moves the first object part in accordance with the moving instructions.

In step (S4) 204, the muscular activity of the object part is measured by the muscular activity sensor, simultaneous to the measured articular angle position of the object, while the human moves the object part in accordance with the moving instructions.

In step (S5) 205, the articular angle position data and the muscular activity data is communicated in real-time to a computer device such as laptop, mobile phone, and the like via Bluetooth or WiFi, where the data is forwarded to a computer platform where the data is processed. The processing many include utilizing the articular angle data to align the muscular activity data with corresponding muscular activity reference data for each acquired articular angle position measured by the orientation sensor and utilizing the resulting muscular activity data pair for each acquired articular angle position measured by the orientation sensor in calculating a real-time movement and performance analysis indicator for the object part. The performance analysis indicator may include calculating the difference between the first muscular activity data and the corresponding muscular activity reference data for each acquired articular angle position measured by the orientation sensor, where if the difference is above or below a pre-defined threshold biofeedback command is issued. This may be any type of visual feedback presented as an example on a display device where the human and medical expert are able to monitor the muscular activity in real-time. If the muscular activity is above or below a pre-defined threshold, i.e. not as it should be, the biofeedback command may include issuing moving instructions at least partly different from the pre-stored moving instructions. This may be repeated until the difference between the muscular activity data and the corresponding muscular activity reference data is within a threshold window.

In step (S6) 206, another wireless mobile monitoring device (second) is provided configured to be placed on another object part of the human different from the previous object part. More than two such monitoring devices may obviously be used.

In step (S7) 207, the articular angle position of the other object part is measured by the second orientation sensor while the human moves the object part in accordance with the moving instructions.

In step (S8) 208, the articular angle position data and the muscular activity data measured by the second monitoring device is transmitted in real-time to the computer device that forwards the data to the platform computer where the data is stored and processed as discussed above.

The other object part may be the same muscle as the previous object part but on the other side of the human, e.g. biceps on right arm and left arm, or left and right shoulder parts.

In an embodiment, the object part may be the injured object part and the previous object part may be the healthy part, where the muscular activity reference data is the measured muscular activity data from the healthy muscular object part.

In another embodiment, the muscular activity reference data comprises pre-stored data from plurality of healthy muscular objects from other humans.

At the end of the recording, a summary of the results may be presented, e.g. displayed on the display, in a simple format so that the medical or physical therapists can make a decision about the next steps. This could as an example be where the muscle is: Left/right deltoid, left/right upper-trapezius, left/right lower-trapezius, left/right serratus, where the data presented in the overview might be max voltages together with the time.

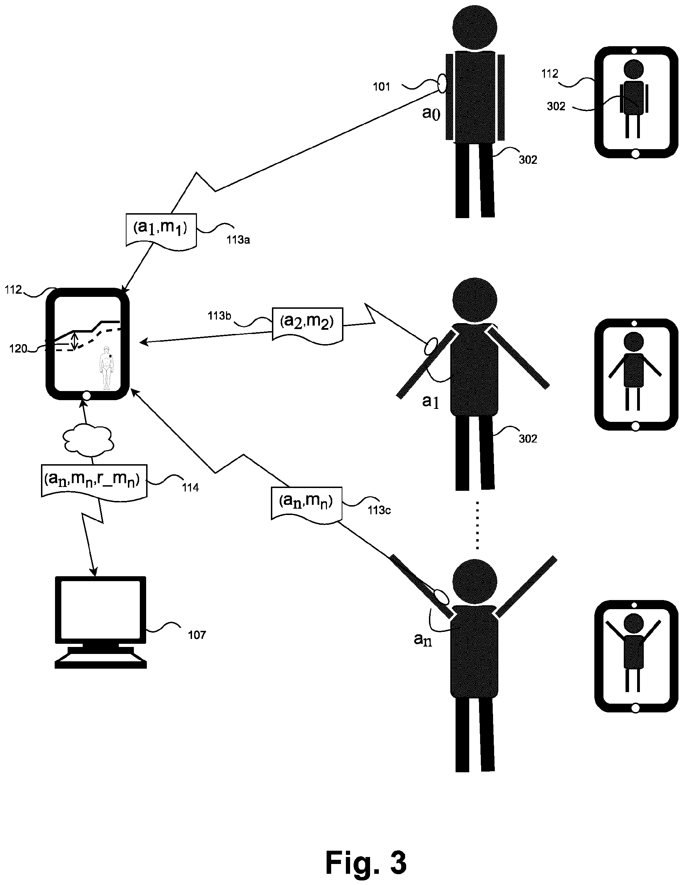

Figure 3 depicts graphically where a human 302 follows moving instructions shown by a silhouette 302 on a display of a computer device, which may be a laptop, tablet, mobile phone 112, PC computer, where the accumulated muscular activity data and the articular angle data 113a,b,c as discussed in relation to figures 1 to 2 for each angular position a₀-aₙ is transmitted in real-time to the computer device 112, that forwards the data to the external computer platform 107, where the processing steps discussed in relation to figure 2 are performed. The processed data 114 is presented on the display of the computer device 112. As discussed previously, the performance analysis indicator 120 may reflect a deviation of the first muscular activity data from the muscular activity reference-data as indicated by the arrow. In that way, the human and/or medical expert may monitor the muscular activity in real-time and adjust the exercises if needed until the deviation is below a pre-defined threshold value.

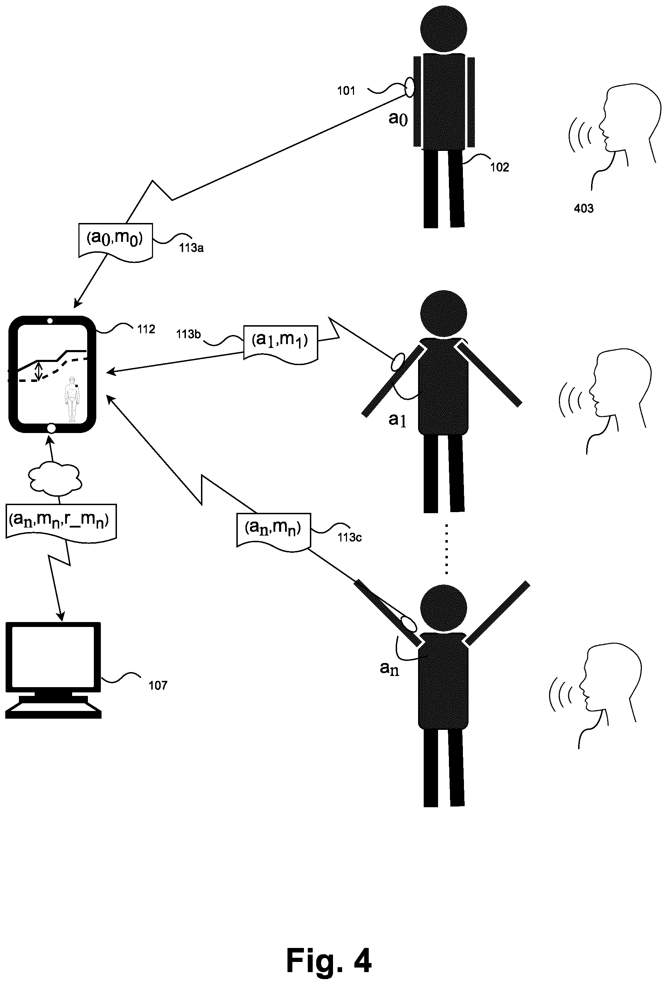

Figure 4 shows the embodiment in figure 3 but where the moving instructions are given orally by a physical therapist or a medical expert 403.

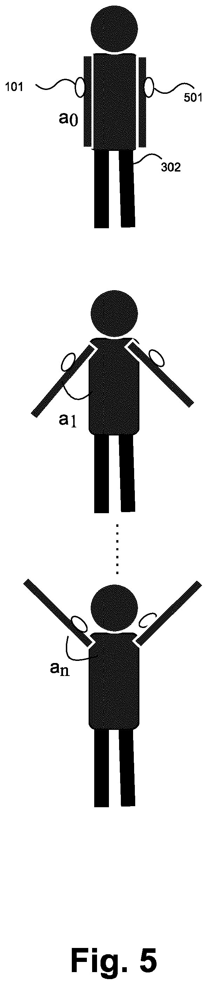

Figure 5 shows an embodiment where a second wireless mobile monitoring device 501, comprising a second transmitter, a second orientation sensor and a second muscular activity sensor. The second wireless mobile monitoring device is placed on the other upper arm on the human 302 on a second object part of the human different from the first muscular object part, which as shown here is the same but on the other arm.

Similar measurements are done where the articular angle position of the second object part is measured together with the muscular activity data while the human moves the second object part in accordance with the moving instructions, where the data is transmitted in real-time to the computer device as discussed previous figures.

The first object part shown in the figures may be seen as injured muscle part, whereas the second object part may be seen as a healthy object part. In that way, the measurements for both the muscular object parts may be compared in real-time.

The movement of the arms does not necessarily need to be done simultaneously as shown here.

It should be noted that the object part may also include muscle or non-muscle object parts on the legs on the human and/or the back and/or the stomach, just to mention few.

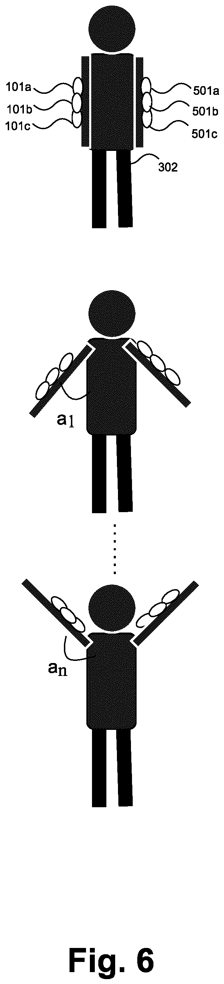

Figure 6 shows another scenario where the three wireless monitoring devices 101a-c and 501a-c are being utilized.

The number of devices by vary depending on the injury, in some instances, 16 devices and even over 30 devices may be used.

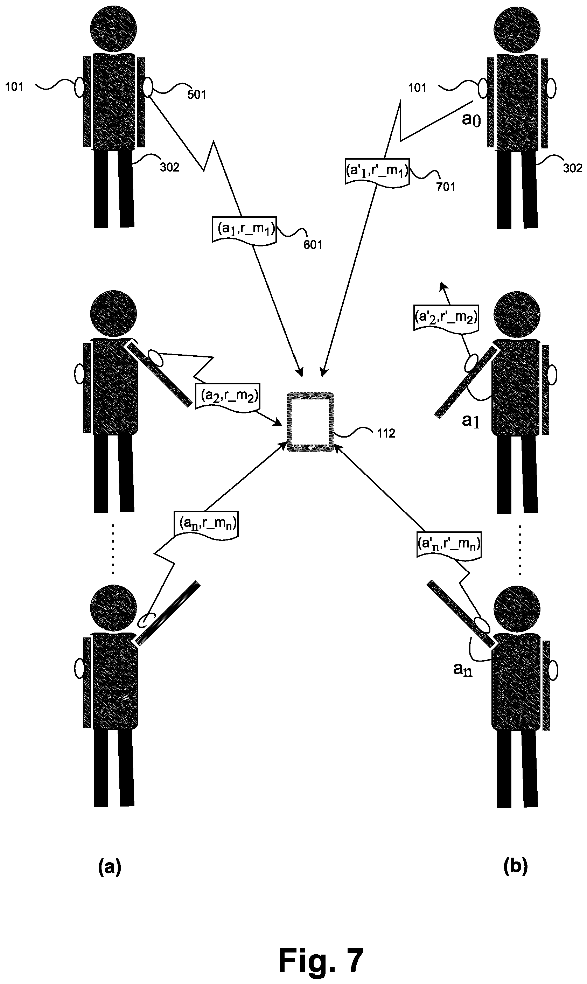

Figure 7(a) shows an exemplary embodiment where the human starts with moving the right healthy arm with the second wireless mobile monitoring device 501 in accordance to the moving instructions as discussed in relation to figures 3 and 4, where for each articular angle position of the second muscular object a₁-aₙ the muscular activity is measured r_m₁ - r_mₙ and transmitted to the external computer system where the measured muscular activity is used as said reference data.

Figure 7(b) shows corresponding data a'₁-a'ₙ and r'_m₁ - r'_mₙ where the movement for the other injured arm is moved according to the moving instruction and transmitted (not shown here) as shown in figure 3 and transmitted to the computer device 112, where the above mentioned processing steps are performed, using the measured data of the healthy arm as reference data.

This issue could also be solved with the same measuring unit by moving it between (then the inactive hand in the image would be without the measuring device). This way, for example, one could compare 4 muscles on the right and 4 muscles on the left side even though using four wireless mobile monitoring devices.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

## Claims

1. A system (100) for real-time movement and performance analysis for a human (102, 302), comprising:
• at least one wireless mobile monitoring device (101) configured to be placed on an object part of the human, comprising:
∘ a communication module (104),
∘ an orientation sensor (106), and/or
∘ a muscular activity sensor (105),
• a computer system configured to present pre-stored moving instructions to the human,
where the orientation sensor and/or the muscular activity sensor is/are configured to acquire articular angle data and/or muscular activity data of the object part while the human moves the object part in accordance with the moving instructions, where the articular angle position data and/or the muscular activity data (113) is/are communicated by the communication module to the computer system where the data is processed, where the processing includes comparing the received articular angle position data and/or the muscular activity data with reference data and determining a real-time movement and performance analysis indicator for the object part.

2. The system according to claim 1, wherein the computer system comprises a computer device comprising a device receiver, a display and a device processor for controlling the device receiver and the display, where the device receiver is configured for receiving the data communicated by the communication module and where the display is configured to display the moving instructions via the display.

3. The system according to claim 1 or 2, wherein the computer system further comprises a computer platform comprising a platform memory, a platform transceiver and a platform processor for controlling the platform memory and the platform transmitter, where the moving instructions are stored by the platform memory, and where the platform processor is configured to instruct the platform transceiver to transmit the stored moving instructions to the computer device where the moving instructions are stored in a device memory comprised in the computer device or displayed in real-time at the computer device via the display.

4. The system according to any of the preceding claims, wherein orientation sensor comprises an Inertial Measurement Unit (IMU) sensor or Magnetic Angular Rate and Gravity (MARG) sensor.

5. The system according to any of the preceding claims, wherein muscular activity sensor comprises a Electromyography (EMG) sensor.

6. The system according to any of the claims 2 to 5, wherein processing the articular angle position data and/or the muscular activity data (113) includes processing both the articular angle position data and the muscular activity data, where the processing is performed by a device processor comprised in the wireless mobile monitoring device(s) or by the platform processor, where the processing comprises:
∘ utilizing the articular angle data to align the muscular activity data with corresponding muscular activity reference data (114) for each acquired articular angle position measured by the orientation sensor, and
∘ utilizing the resulting muscular activity data pair for each acquired articular angle position measured by the orientation sensor in calculating the real-time movement and performance analysis indicator (120) for the muscular object part.

7. A method of real-time movement and performance analysis for a human, where the method comprises:
• providing a pre-stored moving instructions characteristic for an object part, such as muscular or non-muscular object part, of the human,
• presenting the moving instructions to the human,
• providing at least one wireless mobile monitoring device configured to be placed on the object part of the human, comprising:
∘ a communication module,
∘ an orientation sensor, and/or
∘ a muscular activity sensor,
• measuring, by the orientation and/or the muscular activity sensor, the articular angle data and/or muscular activity of the object part while the human moves the object part in accordance with the moving instructions,
• communicating by the communication module the measured articular angle data and/or the muscular activity to a computer system where the data is processed, where the processing includes,
• comparing the received articular angle position data and/or the muscular activity data with reference data and determining a real-time movement and performance analysis indicator for the object part.

8. The method according to claim 7, further comprising initially, in response to selecting an object part or object parts on the human to be analyzed, providing information indicating where to place the orientation and/or the muscular activity sensor on the human.

9. The method according to claim 7 or 8, wherein the step of processing the received articular angle position data and/or the muscular activity data includes processing both the articular angle position data and the muscular activity data, where the step of comparing comprises:
∘ utilizing the articular angle data to align the muscular activity data with corresponding muscular activity reference data (114) for each acquired articular angle position measured by the orientation sensor, and
∘ utilizing the resulting muscular activity data pair for each acquired articular angle position measured by the orientation sensor in calculating the real-time movement and performance analysis indicator (120) for the muscular object part.

10. The method according to any of the claims 7 to 9, wherein multiple of wireless mobile monitoring devices are utilized to be placed on an injured object part and corresponding healthy object part on the human, where the reference data comprises the data measured on the corresponding healthy object.

11. The method according to any of the claims 7 to 10, wherein muscular activity reference data comprises pre-stored data from plurality of healthy muscular objects from other humans.

12. The method according to any of the claims 8 to 11, wherein step of calculating the real-time movement and performance analysis indicator for the object part comprises calculating the difference between the muscular activity data and the corresponding muscular activity reference data for each acquired articular angle position measured by the orientation sensor, where if the difference is above a pre-defined threshold biofeedback command is issued.

13. The method according to claim 12, wherein the biofeedback command comprises issuing moving instructions at least partly different from the pre-stored moving instructions.

14. The method according to claim 13, wherein the biofeedback command is issued until the difference between the first muscular activity data and the corresponding muscular activity reference data is above or below the pre-defined threshold.

15. The method according to any of the claims 9 to 14, wherein the step of processing the received articular angle position data and/or the muscular activity data, and comparing the received articular angle position data and/or the muscular activity data with reference includes:
• processing the received articular angle position data and compare it with articular angle position reference data, or
• processing the received muscular activity data and compare it with muscular activity reference data.
